# EUROPÄISCHE PATENTSCHRIFT

(11) **EP 1 566 161 B1**
(45) Veröffentlichungstag und Bekanntmachung des Hinweises auf die Patenterteilung: **09.11.2011**
(21) Anmeldenummer: 05001302.8
(22) Anmeldetag: 22.01.2005
(51) Int. Cl.: A61K 6/083

(54) **Fliessfähiges Unterfüllungsmaterial**
Flowable liner material
Matériau coulant sous-remplissage

(30) Priorität: 10.02.2004 DE 102004006643
(43) Veröffentlichungstag der Anmeldung: 24.08.2005
(73) Patentinhaber: Heraeus Kulzer GmbH, 63450 Hanau (DE)
(72) Erfinder: Grundler, Andreas, Dr., 42118 Wuppertal (DE); Erdrich, Albert, Dr., 61231 Bad Nauheim (DE); Greczmiel, Michael, Dr., 60385 Frankfurt (DE); Ernst, Claus-Peter, Dr., 55128 Mainz (DE)
(74) Vertreter: Kühn, Hans-Christian

(56) Entgegenhaltungen:
- EP-A- 0 287 213
- EP-A- 0 363 095
- WO-A-00/25729
- GB-A- 2 257 433
- US-A- 4 588 763

## Beschreibung

Die Erfindung betrifft die Verwendung einer Zusammensetzung zur Herstellung eines fließfähigen Unterfüllungsmaterial (Liner), das vor dem Legen von Kompositfüllungen in die präparierte Zahnkavität eingebracht wird.

Adhäsive Restaurationen im Seitenzahnbereich haben bereits seit längerem einen höheren Anteil in der restaurativen Therapie als Amalgam¹ und werden heute in der Regel ohne zusätzliche Unterfüllung verwendet. Nur bei indirekten Restaurationen wie Keramikinlays- und Kronen wird eine rekonstruktive Unterfüllung verfahrenstechnisch nach wie vor als sinnvoll angesehen. Diese kann aber ebensogut aus einem adhäsiv befestigten Stumpfaufbaukomposit bestehen.
¹ Sundberg H, Mejare 1, Espelid 1, Tveit AB: Swedish dentists decisions on preparation techniques and restorative materials. Acta Odontol Scand 58, 135 (2000).

Die Funktion der Unterfüllung - Schutz des Zahnes vor chemischen, thermischen und mechanischen Noxen - übernimmt zum einen das Dentinadhäsiv; vorausgesetzt es wird korrekt angewendet, denn nur dann kann es zum einen postoperative Beschwerden vermeiden und zum anderen das Dentin bakteriendicht versiegeln: Die bakterielle Penetration entlang offener Dentintubuli ist eines der Hauptargumente für das Legen einer Unterfüllung; im ungünstigsten Fall können über die Dentintubuli Bakterien oder Toxine in die Pulpa vordringen und dort zu einer Pulpitis oder einer infizierten Nekrose führen. Dies kann allerdings durch eine mit einem Dentinadhäsiv suffizient versiegelte Oberfläche ebenso effektiv verhindert werden wie mit einer Unterfüllung. Den thermischen und mechanischen Schutz der Dentinwunde übernimmt dann die metallfreie Rekonstruktion aus Komposit oder Keramik.

Ein Argument für die Verwendung von separaten Unterfüllungen, welches sich schwer entkräften lässt, ist das folgende: Müsste eine Kompositfüllung einmal wieder entfernt werden, wird es für den Behandler zu einer wahren Strafarbeit, zahnfarbenes Komposit in tiefen pulpanahen Bereichen oder am cervikalen Kavitätenrand restlos zu entfernen [2]². Oftmals wird hierbei mehr Zahnhartsubstanz entfernt als notwendig [3,4,5]^{3 4 5}, und nicht selten kommt es zu einer iatrogenen Pulpenexposition. Der zudem erhöhte Zeitfaktor bei der Kompositrevision wird von Krejci mit einer mittleren Revisionszeit von 24 Minuten gegenüber z.B. 11 Minuten bei Glasionomerzement und 15 Minuten bei Amalgamfüllungen angegeben [2]. Andere Autoren geben zwar kürzere Revisionszeiten bei Kompositrestaurationen an [6]⁶, beschreiben aber auch eine relative Verlängerung der Behandlungszeit gegenüber anderen, nicht zahnfarbenen Materialien.

² Krejci I, Lieber CM, Lutz F: Time requirement to remove totally bonded tooth-colored posterior restorations and related tooth substance loss. Dent Mater 11, 34 (1995).
Hunter AJ, Hunter AR, Treasure ET: Increases in cavity volume associated with the removal of 3 Class II amalgam and composite restorations. Oper Dent 20, 2 (1995),
⁴ Millar BJ, Robinson PB, Davies BR: Effects of the removal of composite resin restorations on class II cavities. Br Dent J 10, 210 (1992).
⁵ Szep S, Alamouti C, Baum C, Schmidt D, Gerhard T, Heidemann D: Revision von Kompositfüllungen und deren Einfluss auf die Kavitätenimenson und die aufgewendete Zeit in Abhängigkeit vom verwendeten Material. Dtsch Zahnärztl Z 57, 219-226 (2002).
⁶ Ernst CP, Canbek K, Aksogan K, Willershausen B: Two-year clinical performance of a packable posterior composite with and without a flowable composite liner. Clin Oral Invest

Im Allgemeinen und laut Herstellerangaben sollte eine Kompositfüllung überhaupt nicht wieder entfernt werden müssen, da eine lebenslange Haltbarkeit versprochen wird. Die klinische Realität zeigt aber ein anderes Bild: hier muss doch hin und wieder eine Kompositrestauration wieder entfernt werden, auch wenn Kompositmaterialien durchaus auch repariert werden können. Hinsichtlich der Reparaturmöglichkeit sind adhäsive Kompositrestaurationen eindeutig anderen Restaurationsmaterialien überlegen; dennoch bleiben aber einzelne Indikationen, die eine vollständige Entfernung des gesamten Kompositmaterials aus der Kavität erfordern. Nicht immer kann man sich hierbei einfach entlang brauner Randspalte in die Tiefe bewegen; und in tiefen pulpennahen Bereichen besteht stets die Gefahr der Pulpaverletzung. Oft wird auch "zur Sicherheit" deutlich mehr Zahnhartsubstanz entfernt, als eigentlich notwendig wäre: Millar et al. beschreiben, dass sich bei Revisionen von Kompositfüllungen die Kavitäten iatrogen um bis zu 37 % vergrößern [6].

Es hat bereits Versuche gegeben, diesem Problem beizukommen, etwa durch Markieren von Dentalmaterial mit einem photochromen Farbstoff (DE19520016C2). In das Material eingebrachte photochrome Substanzen verfärben sich für kurze Zeit nach oliv-braun, wenn sie mit der Polymerisationslampe belichtet werden, und der Behandler soll beim Entfernen erkennen, ob er noch im Bereich der Füllung arbeitet. Die Entwicklung führte zu dem Produkt "Tetric® Flow Chroma" der Firma Vivadent, Liechtenstein. Szep et al. [5] untersuchten die Verwendung von Tetric Flow Chroma in einer in vitro-Studie; überraschenderweise fanden sie nur in dem Fall, in dem Tetric Ceram Chroma auch an den Kavitätenwänden und nicht ausschließlich auf dem Kavitätenboden aufgebracht wurde eine signifikante Reduktion des Zahnsubstanzverlustes bei der Revision der Füllungen. Auch konnte keine signifikante Reduktion der Revisionszeit - weder bei Verwendung von Tetric Flow Chroma ausschließlich auf dem Kavitätenboden, noch bei Verwendung des Materials an allen Kavitätenrändern - festgestellt werden. Dies mag zum Teil an der erforderlichen, zusätzlichen Lichtaktivierung der photochromen Substanzen mit der Polymerisationslampe liegen. Die Belichtung musste mehrfach erfolgen, der Farbumschlag blieb nicht allzu lang bestehen und konnte zudem bei der normalen Routinekontrolle von Restaurationen durch eine dünnere Füllung hindurch aktiviert werden. Dies gab wiederum dem Behandler durchaus ein "ungutes Gefühl", wenn unter den Restaurationen ein bräunliches Schimmern zu bemerken war. Da man natürlich immer nur Restaurationen anderer entfernen muss, aber selbstverständlich nie die eigenen, blieb immer die Frage unbeantwortet im Raum stehen, ob es sich hierbei um eine unter der Füllung befindliche Karies handelt oder um die Verwendung von "Tetric Flow Chroma". Es besteht also ein Bedarf für geeignete Unterfüllungsmaterialien, die keinen unbegründeten Kariesverdacht verursachen.

Vorgeschlagen wurden ferner Adhäsive mit Farbwechseleigenschaften unter Lichteinfluss (US 6,528,555 B1), wobei der Farbwechsel permanent ist. Dentalmaterialien mit reversiblem Farbwechsel werden auch in US 6,670,436 B2 beschrieben.

In WO0025729A1 werden Füllstoffe besonderer Gestalt für Dentalmaterial und ähnliches Material empfohlen. Das Material kann daneben fachübliche Glasfüller, Pigmente und röntgenopake Zusätze enthalten. Die besonders gestalteten Füllstoffe 3-40 TiO₂ aufweisen.

EP363095A2 beschreibt Fluorid abgebende Materialien, die u.a. als Unterfüllunasmaterial vorgeschlagen werden. Beispiel 9 beschreibt ein Versiegelungsmaterial für Zahnschmelz, das 0,77 Gew-% TiO₂ aufweist. Ein ähnliches Fluorid abgebendes Material wird in GB2257433A beschrieben.

Es stellt sich die Aufgabe, ein fließfähiges, lichthärtendes Unterfüllungsmaterial für Kompositfüllungen bereitzustellen, das nach Aushärtung beim oder nach dem Entfernen einer darüberliegenden Kompositfüllung als solches gut sichtbar und von der Zahnhartsubstanz unterscheidbar ist, ohne dass weitere Maßnahmen wie zusätzliche Belichtung zu ergreifen sind.

Es gilt dabei einen Kompromiss zwischen den Zielen satter Opazität, weißer Farbe und Durchhärtung von mehr als 1 mm (bestimmt nach ISO-Norm 4049) bei Lichtpolymerisation zu finden. Diese 1 mm stammen aus der Vorgabe der ISO-Norm 4049 für opake Füllungsmaterialien.

Wichtig ist es, eine hohe Opazität des hochweiß eingefärbten Flow-Komposites zu realisieren, da ein solches Material nur in einer dünnen Schicht zur Anwendung kommt. Hätte ein derartiges Demarkationsmaterial dieselbe Opazität wie ein konventionelles Flow Komposit, wäre es trotz seiner weißen Farbe nicht oder nur ungenügend auf der Zahnhartsubstanz zu erkennen. Die Einstellung einer entsprechend hohen Opazität ist technisch im Prinzip kein Problem, die anschließende Lichthärtung des Materials hingegen schon. Je opaker ein lichthärtendes Material ist, desto geringere Schichtstärken können in einem Polymerisationszyklus polymerisiert werden.

Überraschenderweise gelingt die Lösung des oben beschriebenen Problems durch die maßvolle Verwendung einer hochweißen Kontrastfärbung in einem fließfähigen Unterfüllungskomposit zur farblich kontrastreichen Markierung des Überganges zur natürlichen Zahnhartsubstanz.

Dabei entsteht ein fließfähiges, als solches ohne weiteres sichtbares und von der Zahnhartsubstanz unterscheidbares, lichthärtendes Unterfüllungsmaterial, enthaltend
A) mindestens ein polymerisierbares Monomer,
B) mindestens einen Polymerisations-Initiator,
C) 0,3-10% Weißpigment,
wobei 0.3 bis 0.5 Gew.-% Titandioxid als Weißpigment enthalten sind.

Der Vorteil bei der Verwendung von hochweißen Pigmenten ist die permanente Färbung, die keine separate Lichtaktivierung o.ä. benötigt. Die farbliche Differenzierung von natürlicher Zahnhartsubstanz ist eine Methode, die bei Stumpfaufbaukompositen bereits seit längerem erfolgreich angewendet wird. Die Kontrastfarben blau, grau oder auch schneeweiß erlauben sehr schnell die Identifizierung verbliebenen Materials an der Präparationsgrenze. Eine derartige blaue Kontrastfarbe ist im lichthärtenden Stumpfaufbaukomosit Rebilda LC (VOCO, Cuxhaven) enthalten. Damit konnte man signifikant reduzierte Revisionszeiten gegenüber allen anderen Vergleichsgruppen (Tetric Ceram mit Tetric Flow, Tetric Flow Chroma auf dem Kavitätenboden und mit Tetric Flow Chroma auf alle Kavitätenflächen) z.T. auch reduzierten Substanzverlust (Ausnahme: kein signifikanter Unterschied zur Gruppe, in der Tetric Flow Chroma auch an den Kavitätenwänden aufgebracht wurde) erzielen [5].

Weiß als Kontrastfarbe der Unterfüllung hat für die Füllungstherapie folgende Vorteile: Es täuscht keine tiefe Karies unter Restaurationen vor, und es stört das gesamtästhetische Erscheinungsbild am wenigsten, wenn nämlich ein derartig eingefärbtes Material an den Kavitätenwänden, z.B. auch auf der cervikalen Stufe des approximalen Kastens aufgebracht wird.

Die Befürchtung, ein hochweißes Unterfüllungsmaterial könnte am Kavitätenrand unästhetische weiße Streifen oder Bänder verursachen, hat sich in der Praxis nicht bestätigt. Der hohe Weißgrad der Unterfüllung kann im Gegenteil ein Vorteil sein, wenn etwa durch Amalgamreste verfärbte Zahnsubstanz eine optische Aufhellung erfährt.

Als Weißpigmente kommen bekannte Oxide mit hohem Brechungsindex in Frage, wie z.B. Al₂O₃, ZrO₂, ZnO oder TiO₂. Bevorzugt ist TiO₂. Die Weißpigmente sind zu 0,3-10% in der Mischung vorhanden, wobei 0,3-0,5 Gew.-% Titandioxid als weißpigment enthalten sind.

Als Monomere kommen die auf dem Dentalgebiet üblichen Monomere in Frage. Bevorzugt sind davon die Methacrylate oder Acrylate. Beispiele sind radikalisch polymerisationsfähige monofunktionelle Monomere wie Mono(meth)acrylamide und Mono(meth)acrylate, Acrylamid, Methacrylamid, N-Ethylacrylamid, Methyl-, Ethyl-, Butyl-, Benzyl-, Furfuryl- oder Phenyl(meth)acrylat, Polyfunktionelle Monomere sind die bekannten polyfunktionellen Acrylate bzw. Methacrylate, wie z.B. Bisphenol-Adi(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- oder Tetraethylenglycoldi(meth)acrylat, Decandioldi(meth)acrylat, Trimethylolpropantri(meth)acrylat, Pentaerythrittetra(meth)acrylat sowie Butandioldi(meth)acrylat, 1,10-Decandioldi(meth)acrylat oder 1,12-Dodecandioldi(meth)acrylat.

Als Füllstoffe kommen neben den erwähnten Weißpigmenten weitere Metalloxide wie Alumina, Zirconia, Zinnoxid, Titania, Metallsulfate, Dentalgläser, pyrogene oder Fällungskieselsäuren, Aluminosilicatgläser, Fluoroaluminosilicatgläser, Quarz, Strontiumsilicat, Strontiumborosilicat, Lithiumsilicat, Lithiumaluminiumsilicat, amorphe Kieselsäuren, Schichtsilikate, Zeolithe oder deren Mischungen in Frage.

Bevorzugte Photoinitiatoren sind Benzophenon, Benzoin sowie deren Derivate oder alpha - Diketone oder deren Derivate wie 9,10-Phenanthrenchinon, Diacetyl oder 4,4-Dichlorbenzil. Besonders bevorzugt werden Campherchinon und 2,2-Dimethoxy-2-phenylacetophenon und besonders bevorzugt alpha -Diketone in Kombination mit Aminen als Reduktionsmittel, wie z.B. 4-(N,N-Dimethylamino)-benzoesäureester, N,N-Dimethylaminoethylmethacrylat, N,N-Dimethylsym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-N-propylphenylphosphinoxid besonders geeignet.

Das erfindungsgemäße hergestellte fließfähige Unterfüllungsmaterial hat zweckmäßig im Strukturabbau/Strukturaufbauversuch mittels Rotationsviskosimetrie (Paar Physika Rheometer UDS 200, Methode Kegel/ Platte, Messkegel MK 20 (25 mm, 1°) glatt; Messplatte 180° glatt, Temperatur 23+/- 0,1 °C) eine Scher-Viskosität von 6,0 bis 100 Pas (Mittel gemessen bei 100/sec Schergeschwindigkeit, Messung nach 30 s Scherung) und 125 bis 400 Pas (Mittel bei 0,05 /sec Schergeschwindigkeit, gemessen 10 sec nach Senken der Schergeschwindigkeit von 100/sec auf 0,05/sec). Bei der Kegel/Platte-Messung befindet sich die Probe in einem Scherspalt zwischen einem sehr flachen Kegel und einer koaxialen Platte. Durch die Wahl des Kegelwinkels wird eine gleichmäßige Schergeschwindigkeitsverteilung im Messspalt erzeugt.

Die Ausführung der Erfindung wird anhand der folgenden Beispiele näher erläutert. Prozentangaben beziehen sich wie auch in der übrigen Beschreibung auf das Gewicht:

### Beispiel 1

Eine Zusammensetzung für ein erfindungsgemäßes Unterfüllungsmaterial hat folgende Komponenten:

| | |
|---|---|
| Bisphenol-A-propyloxy-dimethacrylat | 25,5 % |
| TEDMA | 11,5 % |
| Initiatorkomplex | 1 % |
| Ba-Al-Silikatglas (d₅₀ = 0,985 µ) | 60,5 % |
| Titandioxid silanisiert | 0,5 % |

Die Komponenten werden zusammengegeben und unter Lichtschutz in einem Mischer bis zur Homogenität verarbeitet.

### Beispiel 2

Der Vergleich von Proben mit verschiedenem Weißgrad zeigt dessen Auswirkung auf die Aushärtungstiefe:

| Probe | 1 | 2 | 3 | 4 | 5 | 6 | ISO 4049 |
|---|---|---|---|---|---|---|---|
| Titanweiß (TiO₂) % | 0,05 | 0,06 | 0,11 | 0,25 | 0,33 | 0,50 | keine Vorgaben. |
| Aushärtetiefe bei Belichtung 40 sec mit Translux CL bestimmt nach ISO 4049 | > 3 mm | > 3 mm | > 3 mm | 2,6 mm | 2,2 mm | 2 mm | 1,0 mm für opake Füllungsmaterialien |
| Transparenz [%] | 42,9 | 43,1 | 35 | 23,9 | 18 | 11,2 | keine Vorgaben. |

Es wurden verschieden stark pigmentierte Pasten gemäß vorstehender Tabelle geprüft.

Die Transparenz/Transluzenz-Messungen wurden jeweils mit einem Datacolor Farbmessgerät SF 600, Prüfkörperdicke 1,0 mm, Durchmesser 20 mm, Blende 18 mm, Transparenzmessung durch Messung über weißem und schwarzem Hintergrund (Referenzkachel) bestimmt. Bestimmung der Aushärtetiefe nach ISO 4049, Belichtung mit einer Translux Energy Lampe.

Als idealer Kompromiss zwischen ausreichender Deckkraft - um beim Exkavieren deutlich sichtbar zu sein und um sklerotisiertes Dentin abzudecken - und einer Aushärtetiefe deutlich größer als die ISO 4049 Vorgabe für Füllungsmaterialien zeigt die Probe 6 mit einer Aushärtungstiefe von 2 mm. Da insbesondere bei tiefen Kavitäten Patientenmund das Lichtgerät nicht direkt auf der Oberfläche des zu polymerisierenden Materiales aufgesetzt werden kann ist diese um den Faktor 2 höhere Aushärtetiefe in der Praxis sinnvoll.

Proben mit einem deutlich höheren Gehalt an Titandioxid zeigen zwar ebenfalls eine gute Deckkraft, die Aushärtetiefe sinkt aber weiter. Eine beliebig höhere Initiatorkonzentration könnte dieses zwar wieder z.T. aufwiegen, die Pasten hätten dann aber eine höhere Lichtempfindlichkeit und wären potentiell stärker gelblich verfärbt.

## Patentansprüche

1. Verwendung einer Zusammensetzung enthaltend
**A)** mindestens ein polymerisierbares Monomer
**B)** mindestens einen Photointiator,
**C)** 0,3-10 Gew.-% Weißpigment,
wobei 0.3 bis 0.5 Gew.-% Titandioxid als WeißDiament enthalten sind,
zur Herstellung eines fließfähigen, als solches ohne weiteres sichtbaren und von der Zahnhartsubstanz unterscheidbaren, lichthärtenden Unterfüllungsmaterials, das bei Lichthärtung eine Durchhärtungstiefe von mehr als 1 mm (bestimmt nach ISO 4049) bei 40 s Lichtpolymerisation mit einer Standardpolymerisationslampe aufweist.

2. Verwendung nach Anspruch 1, wobei in Komponente C) mindestens ein Weißpigment aus der Gruppe bestehend aus Al₂O₃, ZrO₂ und ZnO enthalten ist.

3. Verwendung nach Anspruch 1 oder 2, wobei in Komponente C) 0.33 bis 0.5 Gew.% Titandioxid enthalten sind.

4. Verwendung nach Anspruch 1 oder 2, wobei Komponente C) aus 0.3 bis 0.5 Gew.-% Titandioxid als Weißpigment besteht.

5. Verwendung nach Anspruch 3, wobei Komponente C) aus 0.33 bis 0.5 Gew.-% Titandioxid als Weißpigment besteht.

6. Verwendung nach einem der vorstehenden Ansprüche, wobei Komponente C aus 0.5 Gew.% Titandioxid als Weißpigment besteht.

7. Verwendung nach einem der Ansprüche 1-6, wobei Komponente A) ein (Meth)-Acrylat oder Acrylat ist.

8. Verwendung nach einem der vorstehenden Ansprüche, wobei
D) weitere Füllstoffe aus der Gruppe: Dentalgläser, Metalloxide, -sulfate, -silikate, Kieselsäuren oder solche zur Erhöhung der Röntgenopazität enthalten sind.

9. Verwendung, nach Anspruch 8, wobei in Komponente
D) ein Bariumsilikatglas enthalten ist.

10. Verwendung nach einem der vorstehenden Ansprüche zur Herstellung eines Unterfüllungsmaterials mit einer Scher-Viskosität von 6,0 bis 100 Pas (Mittel gemessen bei 100/sec Schergeschwindigkeit, Messung nach 30 s Scherung) und 125 bis 400 Pas (Mittel bei 0,05 /sec Schergeschwindigkeit, gemessen 10 sec nach Senken der Schergeschwindigkeit von 100/sec auf 0,05/sec), bestimmt im Strukturabbau/Strukturaufbauversuch mittels Rotationsviskosimetrie (Kegel/Platte, Messkegel 25 mm, 1°, glatt; Messplatte 180°, glatt, Temperatur 23+/- 0,1 °C).

## Claims

1. Use of a composition containing:
A) at least one monomer that can be polymerised;
B) at least one photoinitiator;
C) 0.3 - 10 % by weight white pigment;
whereby 0.3 to 0.5 % by weight titanium dioxide are contained therein as white pigment,
for the manufacture of a flowable, as such easily visible, light-curing lining material that can be distinguished easily from the tooth structure and has a curing depth after curing with light in excess of 1 mm (determined according to ISO 4049) after 40 s of photopolymerisation using a standard polymerisation lamp.

2. Use according to claim 1, whereby component C) contains at least one white pigment from the group consisting of A1₂O₃, ZrO₂, and ZnO.

3. Use according to claim 1 or 2, whereby component C) contains 0.33 to 0.5 % by weight titanium dioxide.

4. Use according to claim 1 or 2, whereby component C) consists of 0.3 to 0.5 % by weight titanium dioxide as white pigment.

5. Use according to claim 3, whereby component C) consists of 0.33 to 0.5 % by weight titanium dioxide as white pigment.

6. Use according to any one of the preceding claims, whereby component C consists of 0.5 % by weight titanium dioxide as white pigment.

7. Use according to any one of the claims 1-6, whereby component A) is a (meth)acrylate or acrylate.

8. Use according to any one of the preceding claims, whereby D) contains further filling agents from the group of: dental glasses, metal oxides, metal sulfates, metal silicates, silicic acids or agents increasing the radiopacity.

9. Use according to claim 8, whereby_component D) contains a barium silicate glass.

10. Use according to any one of the preceding claims for the manufacture of a lining material having a shear viscosity of 6.0 to 100 Pas (mean measured at a shear rate of 100/sec, measurement after 30 s of shearing) and 125 to 400 Pas (mean at a shear rate of 0.05/sec, measurement 10 sec after decreasing the shear rate from 100/sec to 0.05/sec) in the test of decomposition and recovery of internal structure through rotation viscosimetry (cone/plate, measuring cone 25 mm, 1°, smooth; measuring plate 180°, smooth, temperature 23+/-0.1°C).

## Revendications

1. Utilisation d'une composition contenant
A) au moins un monomère polymérisable,
B) au moins un photo-initiateur,
C) 0,3 à 10 % en poids de pigment blanc,
dans laquelle 0,3 à 0,5 % en poids de dioxyde de titane est contenu en tant que pigment blanc,
pour la fabrication d'un matériau d'obturation fluide, durcissant à la lumière, visible d'emblée en tant que tel et pouvant être différencié de la substance dure de la dent, qui présente lors du durcissement à la lumière une profondeur de durcissement à coeur de plus de 1 mm (déterminée selon ISO 4049) en cas de photo-polymérisation de 40 s avec une lampe de polymérisation standard.

2. Utilisation selon la revendication 1, dans laquelle au moins un pigment blanc parmi le groupe constitué de Al₂O₃, ZrO₂ et ZnO est contenu dans le composant C).

3. Utilisation selon la revendication 1 ou 2, dans laquelle 0,33 à 0,5 % en poids de dioxyde de titane est contenu dans le composant C).

4. Utilisation selon la revendication 1 ou 2., dans laquelle le composant C) se compose de 0,3 à 0,5 % en poids de dioxyde de titane en tant que pigment blanc.

5. Utilisation selon la revendication 3, dans laquelle le composant C) se compose de 0,33 à 0,5 % en poids de dioxyde de titane en tant que pigment blanc.

6. Utilisation selon l'une quelconque des revendications précédentes, dans laquelle le composant C se compose de 0,5 % en poids de dioxyde de titane en tant que pigment blanc.

7. Utilisation selon l'une quelconque des revendications 1 à 6, dans laquelle le composant A) est un (méth)acrylate ou àcrylate.

8. Utilisation selon l'une quelconque des revendications précédentes, dans laquelle D) d'autres charges parmi le groupe : verres dentaires, oxydes métalliques, sulfates métalliques, silicates métalliques, acides siliciques ou similaires pour l'augmentation de l'opacité aux rayons X sont contenues.

9. Utilisation selon la revendication 8, dans laquelle un verre de silicate de baryum est contenu dans le composant D).

10. Utilisation selon l'une quelconque des revendications précédentes pour la fabrication d'un matériau d'obturation avec une viscosité au cisaillement de 6,0 à 100 Pas (moyenne mesurée à une vitesse de cisaillement de 100/s, mesure après cisaillement de 30 s) et 125 à 400 Pas (moyenne à une vitesse de cisaillement de 0,05/s, mesurée 10 s après diminution de la vitesse de cisaillement de 100/s à 0,05/s), déterminée dans l'essai de dégradation de structure/construction de structure au moyen d'une viscosimétrie de rotation (cône/plaque, cône de mesure 25 mm, 1°, lisse ; plaque de mesure 180°, lisse, température 23+/- 0,1 °C).
